# EUROPEAN PATENT APPLICATION

(11) **EP 0 958 804 A2**
(43) Date of publication of application: **24.11.1999**
(21) Application number: 99401115.3
(22) Date of filing: 06.05.1999
(51) Int. Cl.: A61K 7/00, A61K 7/06

(54) **Hair-care treatment composition**

(30) Priority: 20.05.1998 JP 15536898
(71) Applicant: SHIN-ETSU CHEMICAL CO., LTD., Chiyoda-ku Tokyo (JP)
(72) Inventor: Kuwata, Satoshi, Shin-Etsu Chemical Co., Ltd., Usui-gun, Gunma-ken (JP); Inokuchi, Yoshinori, Shin-Etsu Chemical Co., Ltd., Usui-gun, Gunma-ken (JP)
(74) Representative: Armengaud Ainé, Alain

(57) **Abstract**

Disclosed is a novel hair-care treatment composition which can be in a variety of preparation forms such as hair sprays and hair rinse compositions capable of imparting users thereof with an excellent feeling of use not only during but also after the hair-care treatment by using the same. Characteristically, the hair-care treatment composition of the invention is compounded, besides various kinds of base ingredients acceptable for toiletry preparations, with a limited amount of a powder of composite silicone particles, each particle consisting of a core of a cured silicone rubber and a cladding layer of a polyorganosilsesquioxane resin. The composite silicone particles can be prepared by the hydrolysis-condensation reaction of an organotrialkoxy silane in an aqueous suspension of spherical particles of a cured silicone rubber prepared by the hydrosilation reaction between a vinyl-containing organopolysiloxane and an organohydrogen polysiloxane as jointly emulsified in an aqueous medium.

## Description

### BACKGROUND OF THE INVENTION

The present invention relates to a novel hair-care treatment composition or, more particularly, to a hair-care treatment composition containing specific composite silicone particles and capable of imparting the users thereof with a pleasant use feeling of placidness, non-stickiness and refreshingness and imparting the hair treated by using the same with moderate glossiness and smoothness in combing.

While it is conventional that a hair-care treatment composition is formulated with a variety of oily materials in an object that the hair treated by using the composition is imparted with smoothness and lubricity, silicone oils, i.e. organopolysiloxane fluids, are the most widely employed oily materials because the hair having high smoothness after treatment with a silicone oil-containing hair-care treatment composition is free from stickiness along with the very wide availability in the selection of silicone oils relative to the viscosity and vaporizability ranging from a silicone oil of low viscosity having vaporizability to a practically non-vaporizable organopolysiloxane having gum-like consistency.

A problem encountered in the use of a silicone oil as an ingredient in a hair-care treatment composition is that the amount of the silicone oil formulated in the composition is limited because a hair-care treatment composition containing a large amount of a silicone oil is sometimes responsible for the appearance of stickiness of the hair, loosened hair condition and loss of opulent hair voluminosity along with a problem of accumulation of remnant silicone oils on the hair even by repeated shampooing. A further attempt has been made to compound a hair-care treatment composition with an inorganic or organic powder of fine particles but such a powder-containing hair-care treatment composition is not always acceptable due to the problem of loss of glossiness on the hair treated therewith.

### SUMMARY OF THE INVENTION

In view of the above described problems in the conventional hair-care treatment compositions formulated with a silicone oil and/or inorganic or organic fine particles, the present invention accordingly has an object to provide a novel and improved hair-care treatment composition capable of imparting the hair treated by using the composition with moderate glossiness, wettish but non-sticky and smooth touch feeling and smoothness in combing. In this regard, the inventors previously conducted studies on the application of a powder of composite silicone particles (see Japanese Patent Kokai 9-20631).

Thus, the hair-care treatment composition of the present invention comprises, as a uniform blend:
(A) from 0.01 to 50% by weight or, preferably, from 0.05 to 10% by weight of a powder of spherical composite silicone particles, each particle consisting of a core of a cured silicone rubber having an average particle diameter in the range from 0.1 to 100 µm and a cladding layer of a polyorganosilsesquioxane resin formed on the core; and
(B) the balance to 100% by weight of materials acceptable as base ingredients in a hair-care treatment composition.

### DETAILED DESCRIPTION OF THE PREFERRED EMBODIMENTS

As is described above, the essential ingredients in the inventive hair-care treatment composition include the above mentioned components (A) and (B), of which the most characteristic ingredient is the component (A) which is a powder of spherical composite silicone particles, each particle consisting of a core of a cured silicone rubber and a cladding layer of a polyorganosilsesquioxane resin formed on the core.

The spherical composite silicone particles as the component (A) can be prepared by a process described below.

In the first place, an oil-in-water aqueous emulsion is prepared by emulsifying a vinyl group-containing organopolysiloxane and an organohydrogenpolysiloxane in combination in an aqueous medium containing an emulsifying agent with admixture of a catalytic amount of a platinum compound as a catalyst for the hydrosilation reaction to form spherical fine particles of a cured silicone rubber as dispersed in the aqueous medium. The spherical particles of the cured silicone rubber obtained in this way should have a particle diameter in the range from 0.1 to 100 µm when to be used in the present invention.

In the next place, the aqueous suspension of the cured silicone rubber particles is admixed with an organotrialkoxy silane compound such as methyl trimethoxy silane and an alkaline compound as a catalyst to effect hydrolysis-condensation reaction of the organotrialkoxysilane compound in the presence of the cured silicone rubber particles depositing a cladding layer of a polyorganosilsesquioxane resin on the particles of the cured silicone rubber as the core followed by separation of the particles from the aqueous medium and drying thereof to give fine spherical particles suitable as the component (A), each particle consisting of a core of the cured silicone rubber and a cladding layer of the polyorganosilsesquioxane resin on the core. The amount of the cladding layer in the composite silicone particles as the component (A) is preferably in the range from 1 to 20% by weight. This process is known in the art and described, for example, in Japanese Patent Kokai 7-196815.

The amount of the composite silicone particles formulated in the inventive hair-care treatment composition as the component (A) is in the range from 0.01 to 50% by weight or, preferably, from 0.05 to 10% by weight. When the amount thereof is too small, the desired improvements in the hair-care treatment composition cannot be fully obtained as a matter of course while, when the amount thereof is too large, such a composition is no longer suitable for use as a hair-care treatment composition.

It is optional but advantageous that the hair-care treatment composition of the invention is compounded with a silicone of high molecular weight which has an effect of not only to improve the glossiness and combing smoothness of the hair but also to increase sustainability of the improvements.

The component (B) includes a variety of materials acceptable as the ingredients of a hair-care treatment composition including water, alcohols, solid, semi-solid or liquid oily materials, water-soluble polymeric compounds, film-forming agents, surface active agents, inorganic and organic powders, oil-soluble gelation agents, organic-modified clay minerals, resins, ultraviolet absorbers, moisturizing agents, antiseptic or bactericidal agents, perfumes, salts, antioxidants, dandruff preventing agents, antifreeze agents, refrigerant agents, anti-inflammatory agents, chelating agents, skin beautifying agents, vitamin compounds, amino acids, hormones, clathrate compounds and so on.

The above mentioned oily materials include solid, semi-solid and liquid ones originating in plants and animals as well as mineral oils and synthetic oils. Examples of suitable natural oily materials and semi-synthetic oily materials derived therefrom as an oily material include avocado oil, linseed oil, almond oil, insect wax, perilla oil, olive oil, cacao butter, kapok wax, *kaya* oil, carnauba wax, cod liver oil, candelilla wax, beef tallow, hydrogenated beef tallow, apricot kernel oil, spermaceti, hydrogenated oil, wheat germ oil, sesame oil, rice germ oil, rice bran oil, sugar cane wax, *sasanqua* oil, safflower oil, shear butter, Chinese tung oil, cinnamon oil, *jojoba* wax, shellac wax, turtle oil, soybean oil, tea seed oil, *tsubaki* oil, evening primrose oil, corn oil, lard, rape seed oil, Japanese tung oil, bran wax, germ oil, horse oil, persic oil, palm oil, palm kernel oil, castor oil, hydrogenated castor oil, castor oil fatty acid methyl ester, sunflower seed oil, grape seed oil, bayberry wax, *jojoba* oil, macadamia nut oil, beeswax, mink oil, cottonseed oil, cotton wax, japan wax, montan wax, coconut oil, hydrogenated coconut oil, tri(coconut oil fatty acid) glyceride, mutton tallow, peanut oil, lanolin, liquid lanolin, reduced lanolin, lanolin alcohol, hard lanolin, lanolin acetate, lanolin fatty acid isopropyl ester, hexyl laurate, POE lanolin alcohol ethers, POE lanolin alcohol acetate, lanolin fatty acid polyethyleneglycol esters, POE hydrogenated lanolin alcohol ethers and egg yolk oil.

Hydrocarbon oils suitable as the component (B) include ozokerite, squalane, squalene, ceresin, paraffin, paraffin wax, liquid paraffin, pristane, polyisobutylene, microcrystalline wax and Vaselin. Higher fatty acids suitable as the component (B) include lauric acid, myristic acid, palmitic acid, stearic acid, behenic acid, undecylenic acid, oleic acid, linoleic acid, linolenic acid, arachidonic acid, eicosapentaenoic acid, docosahexanoenic acid, isostearic acid and 12-hydroxystearic acid. Higher alcohols suitable as the component (B) include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, POE cholesterol ether and monostearyl glycerin ether.

Ester oils suitable as the component (B) include diisobutyl adipate, 2-hexyldecyl adipate, di-2-heptylundecyl adipate, N-alkylglycol monoisostearates, isocetyl isostearate, trimethylolpropane triisostearate, ethyleneglycol di-2-ethylhexanoate, cetyl 2-ethylhexanoate, trimethylolpropane tri-2-ethylhexanoate, pentaerithritol tetra-2-ethylhexanoate, cetyl octanoate, octyl dodecyl gum ester, oleyl oleate, octyldodecyl oleate, decyl oleate, neopentylglycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerithritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate and diisostearyl malate.

Glyceride oils suitable as the component (B) include acetoglyceride, glycerin triisooctanoate, glycerin triisostearate, glycerin triisopalmitate, glycerin tri-2-ethylhexanoate, glycerin monostearate, glycerin di-2-heptylundecanoate and glycerin trimyristate.

Silicone oils suitable as the component (B) include dimethylpolysiloxanes, methylphenylpolysiloxanes, methylhydrogenpolysiloxanes, octamethyl cyclotetrasiloxane, decamethyl cyclopentasiloxane, dodecamethyl cyclohexasiloxane, tetramethyl cyclotetrasiloxane, higher alcohol-modified silicones such as stearyloxy silicones and higher fatty acid-modified silicones. Fluorocarbon oils suitable as the component (B) include perfluoro polyethers, perfluoro Decalin and perfluorooctane. The above named various oily materials can be used either singly or as a combination of two kinds or more, if compatible.

Examples of the alcohols suitable as the component (B) include lower aliphatic alcohols such as ethyl and isopropyl alcohols, sugar alcohols such as sorbitol and maltose. The sterol compounds include cholesterol, cytosterol, phytosterol and lanosterol.

Examples of the water-soluble polymeric compounds suitable as the component (B) include vegetable-origin polymeric compounds such as gum arabic, tragacanth gum, galactan gum, carob gum, guar gum, karaya gum, carageenan, pectin, agar, quince seed gum, starches of rice, Indian corns, potatoes, wheats and others, algae colloids and locust bean gum, microbe-origin polymeric compounds such as xanthan gum, dextran, succinoglucan and pullulan, animal-origin polymeric compounds such as collagen, casein, albumin and gelatin, starch-based polymeric compounds such as carboxymethyl starch and methyl hydroxypropyl starch, cellulose-based polymeric compounds such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose and cellulose powders, alginic acid-based polymeric compounds such as sodium alginate and alginic acid ester of propyleneglycol, vinylic polymeric compounds such as polyvinyl methyl ether and carboxy divinyl polymer, polyoxyethylene-based polymeric compounds, polyoxyethylene-polyoxypropylene copolymeric compounds, acrylic polymeric compounds such as sodium polyacrylate, polyethyl acrylate and polyacrylamide, cation polymers, inorganic polymeric compounds such as bentonite, aluminum magnesium silicate and anhydrous silicic acid, and so on. Certain film-forming agents such as polyvinyl alcohol and polyvinyl pyrrolidone are also included in this category of water-soluble polymeric compounds.

Surface active agents suitable as the component (B) can be any of anionic, cationic, non-ionic and amphoteric ones selected from those conventionally formulated in cosmetic and toiletry preparations. Examples of the anionic surface active agents include fatty acid soaps such as sodium stearate and triethanolamine palmitate, alkyl ether carboxylic acids and salts thereof, carboxylates as a condensation product of an amino acid and a fatty acid, alkyl sulfonates, alkene sulfonates, sulfonates of a fatty acid ester, sulfonates of a fatty acid amide, sulfonates of a condensation product of an alkyl sulfonate and formaldehyde, sulfates, e,g., alkyl sulfates, secondary higher alcohol sulfates, alkyl ether sulfates, aryl ether sulfates, sulfates of a fatty acid ester, sulfates of a fatty acid alkylolamide and sulfate of Turkey red oil, alkyl phosphates, alkyl ether phosphates, amidophosphates, alkyl aryl ether phosphates and N-acylamino acid-based surface active agents. Examples of the cationic surface active agents include salts of amines such as salts of an alkylamine, polyamines and fatty acid derivatives of an aminoalcohol, alkyl quaternary ammonium salts, aromatic quaternary ammonium salts, pyridinium salts and imidazolium salts.

Examples of the non-ionic surface active agents include sorbitan fatty acid esters, glycerin fatty acid esters, polyglycerin fatty acid esters, propyleneglycol fatty acid esters, polyethyleneglycol fatty acid esters, fatty acid esters of sucrose, polyoxyethylene alkyl ethers, polyoxypropylene alkyl ethers, polyoxyethylene alkylphenyl ethers, polyoxyethylene fatty acid esters, polyoxyethylene-sorbitan fatty acid esters, polyoxyethylene-sorbitol fatty acid esters, polyoxyethylene-glycerin fatty acid esters, polyoxyethylene-propyleneglycol fatty acid esters, polyoxyethylene castor oils, polyoxyethylene hydrogenated castor oils, polyoxyethylene phytostanol ethers, polyoxyethylene phytosterol ethers, polyoxyethylene cholestanol ethers, polyoxyethylehe cholesteryl ethers, polyoxyalkylene-modified organopolysiloxanes, polyoxyalkylene-alkyl-comodified organopolysiloxanes, alkanolamides, sugar ethers and sugar amides. Examples of the amphoteric surface active agents include betaines, aminocarboxylates and imidazoline derivatives.

The powder material suitable as the component (B) is not particularly limitative in respects of the particle configuration which may be spherical, needle-like or platelet-formed, particle diameter and particle structure which may be porous or non-porous and can be selected from those conventionally formulated in toiletry preparations including body pigments, white pigments, blue pigments, organic powders and pearlescent agents exemplified by talc, mica, kaolin, calcium carbonate, magnesium carbonate, titanium dioxide, zinc oxide, red iron oxide, yellow iron oxide, black iron oxide, nylon powders, silk powders, urethane powders, titanium mica and tar dyes. These powder materials can be compounded as such or after a surface treatment with a conventional oiling agent, silicone oil, fluorocarbon compound or surface active agent.

The oil-soluble gelation agent mentioned above is exemplified by metal soaps such as aluminum stearate, magnesium stearate and zinc myristate, amino acid derivatives such as N-lauroyl-L-glutamic acid and α, γ-di-n-butyl amine, dextrin fatty acid esters such as dextrin palmitate, dextrin stearate and dextrin 2-ethylhexanoate palmitate, sucrose fatty acid esters such as sucrose palmitate and sucrose stearate, benzylidene derivatives of sorbitol such as monobenzylidene sorbitol and dibenzylidene sorbitol and so on.

The above mentioned ultraviolet absorber is exemplified by-benzoic acid-based ultraviolet absorbers such as p-amino benzoic acid, anthranilic acid-based ultraviolet absorbers such as methyl anthranilate, salicylic acid-based ultraviolet absorbers such as methyl salicylate, cinnamic acid-based ultraviolet absorbers such as octyl 4-methoxycinnamate, benzophenone-based ultraviolet absorbers such as 2,4-dihydroxy benzophenone, urocanic acid-based ultraviolet absorbers such as ethyl urocanate, and so on.

The moisturizing agent is exemplified by sorbitol, xylitol, propyleneglycol, dipropyleneglycol, 1,3-butyleneglycol, glycerin, diglycerin, polyethyleneglycol, hyaluronic acid, condroitin sulfate and pyrrolidone carboxylates.

The antiseptic agent is exemplified by alkyl paraoxybenzoates, benzoic acid, sodium benzoate, sorbic acid, potassium sorbate and phenoxyethanol. The antimicrobial agent is exemplified by benzoic acid, salicylic acid, phenol, sorbic acid, paraoxybenzoates, p-chloro m-cresol, hexachlorophene, benzalkonium chloride, trichloro carbanilide and phenoxy ethanol.

The antioxidant is exemplified by tocopherol, butyl hydroxyanisole (BHA) and dibutyl hydroxytoluene (BHT). The pH controlling agent is exemplified by lactic acid, citric acid, glycolic acid, succinic acid, tartaric acid, dl-malonic acid, potassium carbonate, sodium hydrogencarbonate and ammonium hydrogencarbonate. The chelating agent is exemplified by alanine, sodium edetate, sodium polyphosphate, sodium metaphosphate and phosphoric acid. The refrigerant agent is exemplified by L-menthol and camphor. The anti-inflammatory agent is exemplified by alantoin, glycyrrhetinic acid, tranexamic acid and azulene.

The dandruff-preventing agent is exemplified by selenium disulfide, brominated alkyl isoquinolinium liquids, zinc pyridione, biphenamine, thiantol and octopirox.

The skin beautifying agents include whitening agents such as placenta extract, arbutin, glutathione and saxifrage extract, cell-invigorating agents such as royal jelly, cholesterol derivatives and juvenile bovine blood extract, rough skin improvers, blood circulation promoters such as benzyl nicotinate, 2-butoxyethyl nicotinate, capsaicine, zingerone, cantharis tincture, ichthammol, caffein, tannic acid, α-borneol, tocopherol nicotinate, inocitol hexanicotinate, cyclandelate, tolazoline, acetyl choline, cepharanthine and γ-orizanol, skin astringents such as zinc oxide and tannic acid, and antiseborrheic agents such as sulfur and thianthol.

The vitamin is exemplified by vitamin A compounds such as vitamin A oil, retinol, retinol acetate and retinol palmitate, vitamin B₂ compounds such as riboflavin, riboflavin butyrate and flavinadenine nucleotide. vitamin B₆ compounds such as pyridoxine hydrochloride and pyridoxine dioctanoate, vitamin C compounds such as L-ascorbic acid, L-ascorbic acid dipalmitate, sodium L-ascorbic acid disulfate and dl- α-tocopherol-L-ascorbic acid phosphoric acid diester dipotassium, pantothenic acid compounds such as calcium pantothenate, D-pantothenyl alcohol, pantothenyl ethyl ether and acetylpantothenyl ethyl ether, vitamin D compounds such as ergocalciferol and cholecalciferol, nicotinic acid compounds such as nicotinic acid, benzyl nicotinate and nicotinic amide, vitamin E compounds such as dl- α -tocopherol, dl- α-tocopherol acetate, dl-α-tocopherol nicotinate and dl- α-tocopherol succinate, vitamin P, biotin and so on.

The amino acid is exemplified by arginine, aspartic acid, cystine, cysteine, methionine, serine, leucine and tryptophane. The nucleic acid is exemplified by deoxy ribonucleic acid. The hormone is exemplified by estradiol and ethenyl estradiol.

The hair-care treatment composition of the present invention can be prepared in a variety of application forms including shampoos, rinses, rinse-in-shampoos, hair treatments, hair conditioners, hair creams, hair oils, hair-brushing agents, hair setting agents, hair restorers and others which can be in a preparation form of a solid, cream, milky lotion, solution, mousse or spray suitable for the particular application.

In the following, the present invention is described in more detail by way of Examples as preceded by a description of the procedure for the preparation of the composite silicone particles although the scope of the invention is never limited by these Examples in any way. The term of "%" appearing in the formulations of the hair-care treatment compositions refers always to "% by weight".

The hair-brushing agents prepared in Example 1 and Comparative Examples 1 and 2 were each subjected to organoleptic eval--uation tests of use for seven items (a) to (g) shown below by 50 female expert panel members. Namely, each of the panel members was requested to report her evaluation of each of the tested hair-care treatment compositions for: (a) dispersibility of the powder in use, (b) stickiness or feeling of heaviness, (c) glossiness of the hair after treatment, (d) smoothness in combing, (e) dry touch feeling of the hair after treatment, (f) touch feeling of lubricity on the treated hair and (g) sustainability of the effects of treatment.

The results of their evaluation for each item were reported in five ratings giving 1 to 5 points according to the following criteria.
5: excellent
4: good
3: fair
2: somewhat poor
1: unacceptable

The values of points given by 50 panel members were averaged for each testing item and the average values for the respective testing items were rated in four ratings of A, B, C and D depending on the average points as follows.
A: 4.5 points or higher
B: 3.5 points or higher but lower than 4.5 points
C: 2.5 points or higher but lower than 3.5 points
D: lower than 2.5 points

### Preparation 1.

Into a glass beaker of 1 liter capacity were taken, under agitation with a homomixer rotating at 2000 rpm, 500 g of a dimethyl polysiloxane terminated at each molecular chain end with a vinyl group and having a viscosity of 600 centistokes at 25 °C as expressed by the structural formula

Vi-SiMe₂-O-(-SiMe₂-O-)₁₈₀-SiMe₂-Vi,

In which Me is a methyl group and Vi is a vinyl group, and 20 g of a methyl hydrogen polysiloxane having a viscosity of 30 centistokes at 25 °C as expressed by the structural formula

Me₃Si-O-(-SiHMe-O-)₁₀-(-SiMe₂-O-)₃₀-SiMe₃,

In which Me is a methyl group, to give a siloxane mixture, which was admixed with 1 g of a polyoxyethylene (9 moles ethylene oxide addition) octylphenyl ether as a surface active agent and 150 g of water and agitated at 6000 rpm to give an oil-in-water emulsion. Thereafter, the oil-in-water emulsion was further admixed with 329 g of water and agitated at 2000 rpm to give a diluted oil-in-water emulsion.

The thus prepared emulsion was transferred into a glass flask of 1 liter capacity equipped with an anchor-blade stirrer and admixed with a mixture of 1 g of a toluene solution of a chloroplatinic acid-olefin complex containing 0.05% of platinum and 1 g of the same surface active agent as used above and kept for 12 hours at room temperature under agitation to effect the hydrosilation reaction giving an aqueous dispersion of spherical cured silicone rubber particles having an average particle diameter of 15 µm as determined by using a Coulter Counter (manufactured by Coulter Electronics Co.).

A 580 g portion of the above obtained aqueous suspension of spherical cured silicone rubber particles was taken in a glass flask of 3 liters capacity together with 2290 g of water and 60 g of a 28% ammonia water to give a reaction mixture into which, under agitation with an anchor-blade stirrer rotating at 200 rpm, 65 g of methyl trimethoxy silane were added dropwise over a period of 20 minutes at 10 °C. After completion of the dropwise addition of the silane compound, agitation of the reaction mixture was further continued first at 5 to 15 °C for 4 hours and then at 55 to 60 °C for 1 hour to complete the hydrolysis-condensation reaction of the silane compound depositing a cladding layer of polymethylsilsesquioxane on the cured silicone rubber particles as the core. The reaction mixture was filtered through a pressurizable filter to give a wet cake of particles containing about 30% of water which was dried in a hot-air circulation oven at 105 °C followed by disintegration of the dried cake in a jet mill to give fine spherical composite silicone particles, referred to as the composite powder 1 hereinafter, each consisting of a core of the spherical cured silicone rubber particle and a cladding layer of a polymethylsilsesquioxane resin derived from methyl trimethoxy silane.

The composite particles as dispersed in water were subjected to the measurement of the average particle diameter by using a Coulter Counter to obtain a value of 15 µm. A gravimetric analysis of the particles indicated that the composite particles consisted of 100 parts by weight of the core particles and 10 parts by weight of the cladding layers.

### Preparation 2.

The procedure for the preparation of spherical composite silicone particles, referred to as the composite powder 2 hereinafter, was substantially the same as in Preparation 1 described above excepting for the replacement of 65 g of methyl trimethoxy silane with a mixture of 55 g of methyl trimethoxy silane and 10 g of 3-glycidyloxypropyl trimethoxy silane. The composite silicone particles had an average particle diameter of 15 µm as determined in the same manner as in Preparation 1. The amount of the cladding layers of the polyorganosilsesquioxane resin was 11 parts by weight per 100 parts by weight of the core particles of the cured silicone rubber.

### Preparation 3.

The procedure for the preparation of spherical composite silicone particles, referred to as the composite powder 3 hereinafter, was substantially the same as in Preparation 1 described above except that the aqueous suspension of spherical cured silicone rubber particles was prepared by increasing the amount of the polyoxyethylene octylphenyl ether as the surface active agent in the preparation of the oil-in-water aqueous emulsion of mixed organopolysiloxanes from 1 g in Preparation 1 to 5 g so that the cured silicone rubber particles in the aqueous suspension had an average particle diameter of 3 µm as determined by using a Coulter Counter. The amount of the cladding layers of the polymethylsilsesquioxane resin was 10 parts by weight per 100 parts by weight of the core particles of the cured silicone rubber.

### Example 1.

A hair-brushing spray was prepared according to the following formulation of 6 ingredients given in % by weight. The results of the organoleptic evaluation tests are shown in Table 1.

| | | |
|---|---|---|
| (1) | Isopropyl myristate | 1.0% |
| (2) | Stearyl trimethyl ammonium chloride | 0.05% |
| (3) | Composite powder 1 | 7.0% |
| (4) | Ethanol | 25.0% |
| (5) | Perfume | q.s. |
| (6) | Propellant | (balance to 100%) |

A mixture of the ingredients (1) to (5) was introduced into a container for aerosol spray followed by filling of the container with the ingredient (6) to give a hair-brushing spray of the invention.

The results of the organoleptic evaluation tests are shown in Table 1.

### Comparative Example 1.

A comparative hair-brushing spray was prepared in substantially the same formulation as in Example 1 excepting for the replacement of the composite powder 1 with the same amount of a polymethylsilsesquioxane powder which was a commercial product consisting of spherical particles having an average particle diameter of 12 µm (Tospearl 3120, a product by Toshiba Silicone Co.).

The results of the organoleptic evaluation tests of this comparative hair-brushing spray are shown in Table 1.

### Comparative Example 2.

Another comparative hair-brushing spray was prepared in substantially the same formulation as in Example 1 excepting for the replacement of the composite powder 1 with the same amount of a cured silicone rubber powder which was a commercial product consisting of spherical particles having an average particle diameter of 10 µm (Torayfil E501, a product by Toray Dow Corning Co.).

The results of the organoleptic evaluation tests of this comparative hair-brushing spray are shown in Table 1.

**Table 1**

| Testing Item | Example 1 | Comparative Example 1 | Comparative Example 2 |
|---|---|---|---|
| (a) | A | B | D |
| (b) | A | B | B |
| (c) | A | C | D |
| (d) | A | B | D |
| (e) | A | B | B |
| (f) | A | D | A |
| (g) | A | C | B |
| overall | A | D | D |

As is understood from the results shown in Table 1, the hair-brushing spray according to the invention was very excellent as compared with the comparative hair-brushing spray of Comparative Example 1 in respects of glossiness and smoothness of the hair and sustainability of the effect obtained by the treatment with the hair-brushing spray and as compared with that of Comparative Example 2 in respects of dispersibility of the powder and combing smoothness and glossiness of the hair treated therewith.

### Example 2.

A hair spray was prepared from 5 ingredients according to the following formulation.

| | | |
|---|---|---|
| (1) | Dimethyl silicone, 1,000,000 cS | 3.0% |
| (2) | Decamethyl cyclopentasiloxane | 75.0% |
| (3) | Composite powder 2 | 7.0% |
| (4) | Perfume | q.s. |
| (5) | Propellant | (balance to 100%) |

A mixture of the ingredients (1) to (4) was introduced into an aerosol container which was then filled with the ingredient (5) to give a hair spray.

The thus prepared hair spray was subjected to the evaluation tests by panel members who reported that the hair spray was excellent in respects of dispersibility of the powder in use, absence of stickiness or feeling of heaviness, adequate gloss of the hair, dry and smooth touch feeling of the hair and wettishness of the hair and combing smoothness of the hair as well as sustainability of these effects.

### Example 3.

A hair rinse composition was prepared from 13 ingredients according to the following formulation.

| | | |
|---|---|---|
| (1) | Ethyleneglycol distearate | 3.0% |
| (2) | Cetanol | 2.0% |
| (3) | Propyleneglycol monostearate | 3.0 % |
| (4) | Dimethylpolysiloxane, 100 cS | 3.0% |
| (5) | Glycerin monostearate | 4.0% |
| (6) | Polyoxyethylene (3 moles EO addition) stearate | 4.0% |
| (7) | Acetyl trimethyl ammonium chloride | 5.0% |
| (8) | Polyoxyethylene (20 moles EO addition) cetyl ether | 2.0% |
| (9) | Composite powder 3 | 4.0% |
| (10) | 1,3-Butyleneglycol | 5.0% |
| (11) | Antiseptic agent | q.s. |
| (12) | Perfume | q.s. |
| (13) | Purified water | (balance to 100%) |

Two separate mixtures were prepared, one, from the ingredients (1) to (9) at room temperature and, the other, from the ingredients (10), (11) and (13) under heating and these mixtures were blended together and cooled to room temperature followed by the addition of the ingredient (12) to give a hair rinse composition.

The thus prepared hair rinse composition was subjected to the organoleptic evaluation tests by female expert panel members who reported that the hair rinse composition was excellent in respects of absence of stickiness or feeling of heaviness in use, moderate glossiness, dry touch feeling, lubricity and voluminosity imparted to the hair and sustainability of the effects obtained by rinsing therewith.

### Example 4.

A rinse-in-shampoo composition was prepared from 11 ingredients according to the following formulation.

| | | |
|---|---|---|
| (1) | Lauroyl amide betain propyl dimethylamino acetate (30%) | 15.0% |
| (2) | Sodium polyoxyethylene (3 moles EO addition) lauryl ether sulfate (27%) | 4.0% |
| (3) | Polyoxyethylene (150 moles EO addition) distearate | 0.5% |
| (4) | Cationated cellulose (4%) | 0.5% |
| (5) | Glycerin | 3.0% |
| (6) | Polydimethylsiloxane, 1,000,000 cS | 1.0% |
| (7) | Polydimethylsiloxane, 6 cS | 3.0% |
| (8) | Composite powder 2 | 2.0% |
| (9) | Antiseptic agent | q.s. |
| (10) | Perfume | q.s. |
| (11) | Purified water | (balance to 100%) |

A mixture of the ingredients (1) to (5), (9) and (11) prepared under heating and a suspension of the ingredients (6) to (8) were admixed together and cooled to room temperature followed by the addition of the ingredient (10) to give a rinse-in-shampoo composition.

The thus prepared rinse-in-shampoo composition was subjected to the evaluation tests by panel members who reported that the rinse-in-shampoo composition was excellent in respects of absence of stickiness or feeling of heaviness in use, moderate glossiness, dry touch feeling, lubricity, voluminosity and combing smoothness imparted to the hair and sustainability of the effects obtained by the treatment therewith.

### Example 5.

A hair treatment composition was prepared from 13 ingredients according to the following formulation.

| | | |
|---|---|---|
| (1) | Ethyleneglycol distearate | 1.0% |
| (2) | Liquid paraffin | 10.0% |
| (3) | Squalane | 5.0 % |
| (4) | Stearyl alcohol | 1.5% |
| (5) | Dimethylpolysiloxane, 10 cS | 3.0% |
| (6) | Stearic acid | 6.0% |
| (7) | Polyoxyethylene (3 moles EO addition) stearyl alcohol | 4.5% |
| (8) | Polyoxyethylene (150 moles EO addition) cetyl ether | 2.0% |
| (9) | Composite powder 3 | 1.5% |
| (10) | 1,3-Butyleneglycol | 6.0% |
| (11) | Antiseptic agent | q.s. |
| (12) | Perfume | q.s. |
| (13) | Purified water | (balance to 100%) |

A mixture of the ingredients (1) to (9) prepared under heating and a mixture of the ingredients (10), (11) and (13) were blended together and cooled to room temperature followed by the addition of the ingredient (12) to give a hair treatment composition.

The thus prepared hair treatment composition was subjected to the evaluation tests by panel members who reported that the hair treatment composition was excellent in respects of absence of stickiness or feeling of heaviness in use, moderate glossiness, dry touch feeling, lubricity, voluminosity and combing smoothness imparted to the hair and sustainability of the effects obtained by the treatment therewith.

## Claims

1. A hair-care treatment composition which comprises, as a uniform blend:
(A) from 0.01 to 50% by weight of a powder of composite silicone particles, each particle consisting of a core of a cured silicone rubber and a cladding layer of a polyorganosilsesquioxane resin formed on the core; and
(B) the balance to 100% by weight of materials acceptable as base ingredients in a hair-care treatment composition.

2. The hair-care treatment composition as claimed in claim 1 in which the content of the component (A) is in the range from 0.05 to 10% by weight.

3. The hair-care treatment composition as claimed in claim 1 in which the polyorganosilsesquioxane is a polymethylsilsesquioxane.

4. The hair-care treatment composition as claimed in claim 1 in which the composite silicone particles each have a spherical particle configuration.

5. The hair-care treatment composition as claimed in claim 1 in which the composite silicone particles have an average particle diameter in the range from 0.1 to 100 µm.

6. The hair-care treatment composition as claimed in claim 1 in which the amount of the cladding layer of a polyorganosilsesquioxane resin on the core is in the range from 1 to 20% by weight based on the amount of the composite silicone particles.

7. The hair-care treatment composition as claimed in claim 1 in which the cured silicone rubber is a product of a hydrosilation reaction between a vinyl group-containing organopolysiloxane and an organohydrogenpolysiloxane mixed together in the form of an oil-in-water emulsion in an aqueous medium.

8. The hair-care treatment composition as claimed in claim 1 in which the polyorganosilsesquioxane resin is a hydrolysis-condensation product of an organotrialkoxy silane compound in an aqueous medium in the presence of particles of a cured silicone rubber.

9. The hair-care treatment composition as claimed in claim 7 in which the organotrialkoxy silane compound is methyl trimethoxy silane.
